# DEMANDE DE BREVET EUROPEEN

(11) **EP 0 694 527 A1**
(43) Date de publication de la demande: **31.01.1996**
(21) Numéro de dépôt: 95401739.8
(22) Date de dépôt: 24.07.1995
(51) Int. Cl.: C07C 227/40, C07C 231/22, C12S 13/00

(54) **Procédé biologique d'élimination des traces de dihalogenoacides, des solutions aqueuses d'amino-acides**

(30) Priorité: 27.07.1994 FR 9409287
(71) Demandeur: RHONE-POULENC CHIMIE, F-92408 Courbevoie Cédex (FR)
(72) Inventeur: Favre-Bulle, F-69007 Lyon (FR); Ricca, Jean-Marc, F-69007 Lyon (FR)
(74) Mandataire: Fabre, Madeleine-France

(57) **Abrégé**

Procédé d'élimination des traces d'acides dihalogénocarboxyliques ou leurs sels, des solutions aqueuses contenant au moins 20% en poids d'amino-acides ou dérivés d'amino-acides et moins de 1000 ppm d'acides dihalogénocarboxyliques ou leurs sels, par traitement direct avec de l'ordre de 1 à 100 ppm d'un micro-organisme contenant une enzyme spécifique des acides dihalogénocarboxyliques ou de leurs sels.

## Description

La présente invention a pour objet un procédé biologique d'élimination des traces d'acides dihalogénocarboxyliques ou de leurs sels, des solutions aqueuses d'aminoacides ou dérivés d'amino-acides, à l'aide d'une faible quantité d'un micro-organisme contenant une enzyme spécifique des acides dihalogénocarboxyliques ou de leurs sels.

Les solutions aqueuses d'amino-acides ou de dérivés d'amino-acides trouvent en particulier des applications dans le domaine de la cosmétologie comme agents tensioactifs ou séquestrants ; ces solutions venant en contact avec les systèmes biologiques, il convient d'éviter la présence de toute impureté susceptible de contribuer aux phénomènes d'irritation.

Parmi les produits pouvant contribuer à ces phénomènes, on peut citer en particulier les acides dihalogénocarboxyliques et leurs sels.

Les amino-acides sont généralement préparés par condensation d'une solution aqueuse d'un dérivé aminé avec un acide halogénocarboxylique ou l'un de ses dérivés. De par leur procédé de préparation, les acides halogénocarboxyliques ou leurs dérivés peuvent contenir jusqu'à 5% de leur poids d'acide dihalogénocarboxylique (notamment dichloro- .ou dibromo- carboxylique)

A titre d'exemple, les acides acétiques α - aminosubstitués de formule 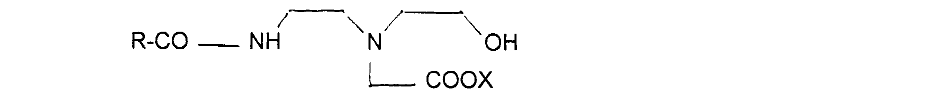
où R représente H, un groupement akyle ou alcoxyle, et où X représente H, un métal alcalin ou alcalino-terreux ou un reste ammonium,
sont classiquement obtenus par condensation d'une amine secondaire de formule 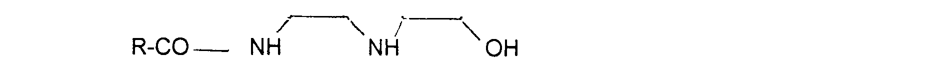
avec l'acide chloroacétique ou l'un de ses sels, notamment le chloroacérate de sodium.

Il a été constaté que les solutions aqueuses obtenues peuvent contenir jusqu'à 500 ppm d'acide dichloroacétique ou de ses sels. Ces teneurs pourraient être abaissées jusqu'à 50 ppm par l'emploi d'un acide chloroacétique purifié, ce qui entraînerait un surcoût incompatible avec les applications de ces produits.

Il a été proposé (WO 93/20223) de décomposer les acides haloalcanoïques présents comme impureté dans les agents tensio-actifs, par contact avec une enzyme déhalogénase.
Il est ainsi décrit d'hydrolyser les acides monochloropropionique ou monochloroacétique ou leurs sels de sodium présents à raison de plusieurs g/l dans des solutions aqueuses à 12% en poids de tensio-actifs (éthers carboxylates notamment) tamponnées à pH 7,2, par le *Pseudomonas putida* NCIB 12018 ; ce dernier est mis en oeuvre à raison de 2g/l, soit 2000 ppm par rapport au milieu.

Les solutions aqueuses obtenues contiennent une quantité de cellules résiduelles incompatibles, tant d'un point de vue des performances que de la tolérance biologique, pour les applications en cosmétologie.

Il a maintenant été trouvé un procédé d'élimination des traces d'acides dihalogénocarboxyliques ou leurs sels, des solutions aqueuses contenant au moins 20% en poids d'amino-acides ou dérivés d'amino-acides et moins de 1000ppm (parties en poids par million), de préférence moins de 200ppm, d'acides dihalogénocarboxyliques ou leurs sels, par traitement direct avec de l'ordre de 1 à 100ppm (parties en poids par million), de préférence de l'ordre de 10 à 50ppm, d'un micro-organisme contenant une enzyme spécifique des acides dihalogénocarboxyliques ou de leurs sels.

Parmi les enzymes spécifiques des acides dihalogénocarboxyliques ou de leurs sels, on peut citer notamment celles capables d'hydrolyser l'acide dichloroacétique ou ses sels (sels de métaux alcalins et d'ammonium).

Ces enzymes sont issues de micro-organismes, qui peuvent être sélectionnées par mise en contact direct avec l'acide dichloroacétique ou ses sels dans les conditions connues de l'homme de l'art (Manual of Methods for General Bacteriology - American Society for Microbiology - 1981)

On peut mentionner tout particulièrement les micro-organismes qui hydrolysent au moins 10% en poids de l'acide dichloroacétique ou de ses sels, tels que notamment les Xanthobacter, et plus particulièrement le *Xanthobacter autotrophicus* ATCC 43050.

Selon le procédé de l'invention, le micro-organisme peut être utilisé tel quel ou immobilisé sur des supports bien connus de l'homme de l'art, comme des gels polyacrylamides, carrageenane, alginates ... ou des résines telles que celles du type Amberlite® en présence d'un agent réticulant du type polyéthylèneimides.

Le micro-organisme peut également être muté par des agents mutagènes ou par génie génétique, suivant de techniques bien connues de l'homme de l'art (Omston et al. - Journal of Biological Chemistry, Vol 241, p. 3800-3810).
Par ailleurs l'information génétique qui code pour l'enzyme, peut être transférée du micro-organisme parental (tel que *Xanthobacter autotrophicus* ) vers un micro-organisme adapté non pathogène et non porteur naturellement de ladite information génétique (tel que *Escherichia coli*, *Bacillus subtilis*)

Une variante du procédé de l'invention, consiste à mettre en oeuvre en quantité correspondante, à la place d'un micro-organisme, son enzyme libre ou immobilisée, celle-ci étant totalement ou partiellement purifiée.

Dans un tel cas, la quantité d'enzyme mise en jeu dans le traitement est comprise entre 0,1 à 10 ppm, de préférence de l'ordre de 1 à 5 ppm.

Ledit procédé de l'invention peut être réalisé à une température de l'ordre de 5 à 50°C, de préférence de l'ordre de 10 à 40°C, en fonction de la thermostabilité de l'enzyme, à un pH de l'ordre de 8 à 12, de préférence de l'ordre de 8,5 à 10.
Ce traitement peut durer de l'ordre de 1 à 24 heures, préférentiellement de 1 à 4 heures.

Parmi les aminoacides ou leurs dérivés susceptibles d'être traités selon le procédé de l'invention, on peut mentionner notamment :
. les agents tensio-actifs de type amphotère, parmi lesquels les alkylbétaïnes, les alkylamidopropylbétaïnes, les dérivés d'imidazolines comme les alkylamphoacétates et diacétates, les alkylhydroxyéthyl- et alkyldihydroxyéthyl- glycinates ...
. les agents séquestrants comme les acides éthylène diamine tétraacétique (EDTA), N(hydroxyéthyl) éthylène diamine triacétique (HEEDTA), nitrilo triacétique (NTA), diethylène triamine pentaacétique (DTPA), ...

agents qui peuvent être obtenus par condensation d'une solution aqueuse d'un dérivé aminé avec un acide halogénocarboxylique ou l'un de ses dérivés.

Le traitement faisant l'objet de l'invention, peut intervenir soit directement à la fin de la réaction de condensation, soit au cours du stockage du produit fini.

Il a été constaté que les teneurs en acide dihalogénocarboxylique sont inférieures à 50ppm, voire même inférieures aux limites de détection des techniques analytiques couramment utilisées par l'homme de l'art, comme par exemple la chromatographie ionique.

Le procédé de l'invention présente de nombreux avantages, à savoir :
- de par les faibles quantités de micro-organismes mises en oeuvre, l'intégrité du produit traité (solutions aqueuses d'amino-acides ou leurs dérivés) est conservée, tant du point de vue de ses performances applicatives que du point de vue tolérance biologique ;
- l'utilisation, pour la préparation dudit produit à traiter, de tout acide halogénocarboxylique du commerce, quelle que soit sa teneur en impureté dihalogénocarboxylique ;
- la possibilité de traiter ledit produit tel quel, sans mise en forme préalable de ce dernier.

Les exemples suivants sont donnés à titre illustratif

### Exemple 1

### Culture du micro-organisme

La souche *Xanthobacter autotrophicus* ATCC 43050 est cultivée en fiole agitée à 30°C pendant 48 heures, soit dans le milieu NB ("nutrient broth") soit dans le milieu A, dont les compositions sont les suivantes :
* milieu NB :

| | |
|---|---|
| Extrait de boeuf | 3 g/l |
| Peptone | 5 g/l |
| NaCl | 8 g/l |
| pH 7,3 | |

* milieu A :

| | |
|---|---|
| Succinate | 5 g/l |
| Dichloroacétate | 10 mM |
| Na₂HPO₄ | 5,7 g/l |
| KH₂PO₄ | 1,4 g/l |
| (NH₄)₂SO₄ | 0,5 g/l |
| MgSO₄, 7 H₂O | 0,2 g/l |
| Extrait de levure | 0,1 g/l |
| FeSO₄, 7 H₂O | 20 mg/l |
| MnSO₄, H₂O | 10 mg/l |
| pH 7,2 | |

la biomasse obtenue est séparée par centrifugation, puis est resuspendue dans une solution de NaCI à 9g/l.

### Traitement

Une solution aqueuse commerciale de cocoylamphodicarboxylate contenant 30% en poids de matières actives, 11% en poids de NaCI, 7% en poids de glycolate de sodium et 270ppm de dichloroacétate de sodium et présentant un pH 8,5, est traitée pendant 24 heures à 25°C, par 50ppm de cellules issues des milieux ci-dessus.
Le dichloroacétate de sodium résiduel est mesuré par chromatographie ionique.
Les resultats obtenus sont les suivants :

| **culture** | **dichloroacétate de sodium résiduel (ppm)** |
|---|---|
| témoin (sans cellules) | 270 |
| NB | 223 |
| A | 33 |

### Exemple 2

### Culture du micro-organisme

La souche *Xanthobacter autotrophicus* ATCC 43050 est cultivée en fiole agitée à 30°C pendant 48 heures, dans le milieu B, dont la composition est la suivante :
* milieu B :

| | |
|---|---|
| Dichloroacétate | 25 mM |
| Na2HPO4 | 5,7 g/l |
| KH2PO4 | 1,4 g/l |
| (NH4)2SO4 | 0,5 g/l |
| MgSO4, 7 H2O | 0,2 g/l |
| Extrait de levure | 0,1 g/l |
| FeSO4, 7 H2O | 20 mg/l |
| MnSO4, H2O | 10 mg/l |
| pH 7,2 | |

la biomasse obtenue est séparée par centrifugation, puis est resuspendue dans une solution de NaCI à 9g/l.

### Traitement

Une solution aqueuse commerciale de cocoylamphodicarboxylate contenant 30% en poids de matières actives, 11% en poids de NaCI, 7% en poids de glycolate de sodium et 270ppm de dichloroacétate de sodium et présentant un pH 8,5, est traitée pendant 24 heures à 25°C, par 10ppm de cellules issues du milieu ci-dessus.
Le taux de dichloroacétate de sodium résiduel, mesuré par chromatographie ionique, est inférieur à 25ppm, limite de détection de l'appareil.

## Revendications

**1)** Procédé d'élimination des traces d'acides dihalogénocarboxyliques ou leurs sels, des solutions aqueuses contenant au moins 20 % en poids d'amino-acides ou dérivés d'amino-acides et moins de 1000 ppm (parties en poids par million), de préférence moins de 200 ppm, d'acides dihalogénocarboxyliques ou leurs sels, par traitement direct avec de l'ordre de 1 à 100 ppm, de préférence de l'ordre de 10 à 50 ppm, d'un micro-organisme contenant une enzyme spécifique des acides dihalogénocarboxyliques ou de leurs sels.

**2)** Procédé d'élimination des traces d'acides dihalogénocarboxyliques ou leurs sels, des solutions aqueuses contenant au moins 20 % en poids d'amino-acides ou dérivés d'amino-acides et moins de 1000 ppm, de préférence moins de 200 ppm, d'acides dihalogénocarboxyliques ou leurs sels, par traitement direct avec de l'ordre de 0,1 à 10 ppm, de préférence de l'ordre de 1 à 5 ppm, d'une enzyme spécifique des acides dihalogénocarboxyliques ou de leurs sels issue d'un micro-organisme.

**3/** Procédé selon la revendication 1), caractérisé en ce que ledit micro-organisme contient une enzyme spécifique de l'acide dichloracétique ou de ses sels.

**4)** Procédé selon l'une des revendications précédentes, caractérisé en ce que ledit micro-organisme est choisi parmi les Xanthobacter.

**5)** Procédé selon la revendication 4), caractérisé en ce que ledit micro-organisme est le *Xanthobacter autotrophicus* ATCC 43050.

**6)** Procédé selon l'une quelconque des revendications précédentes, caractérisé en ce que ledit traitement est réalisé à une température de l'ordre de 5 à 50°C, de préférence de l'ordre de 10 à 40°C, en fonction de la thermostabilité de l'enzyme, à un pH de l'ordre de 8 à 12, de préférence de l'ordre de 8,5 à 10.

**7)** Procédé selon l'une quelconque des revendications précédentes, caractérisé en ce que lesdits aminoacides ou leurs dérivés sont :
. les agents tensio-actifs de type amphotère choisis parmi les alkylbétaïnes, les alkylamidopropylbétaïnes, les alkylamphoacétates et diacétates, les alkylhydroxyéthyl- et alkyldihydroxyéthyl- glycinates .
. les agents séquestrants choisis parmi les acides éthylène diamine tétraacétique (EDTA), N(hydroxyéthyl) éthylène diamine triacétique (HEEDTA), nitrilo triacétique (NTA), diethylène triamine pentaacétique (DTPA),
obtenus par condensation d'une solution aqueuse d'un dérivé aminé avec un acide halogénocarboxylique ou l'un de ses dérivés.
